# EUROPÄISCHE PATENTSCHRIFT

(11) **EP 2 220 996 B1**
(45) Veröffentlichungstag und Bekanntmachung des Hinweises auf die Patenterteilung: **08.02.2012**
(21) Anmeldenummer: 10450014.5
(22) Anmeldetag: 09.02.2010
(51) Int. Cl.: A61B 5/103, G01L 19/08, A61F 13/00

(54) **Druckindikator für medizinische Druckverbände**
Pressure indicator for medical compresses
Indicateur de pression pour pansements compressifs médicaux

(30) Priorität: 20.02.2009 AT 2882009
(43) Veröffentlichungstag der Anmeldung: 25.08.2010
(73) Patentinhaber: IMA Integrated Microsystems Austria GmbH, 2700 Wr. Neustadt (AT)
(72) Erfinder: Frühwirt, Christine, 2700 Wiener Neustadt (AT); Kment, Christoph, 1130 Wien (AT); Schmid, Günther, 1230 Wien (AT); Schnur, Johannes, 7423 Pinkafeld (AT); Weissenböck, Herbert, 2873 Feistritz am Wechsel (AT)
(74) Vertreter: Haffner und Keschmann Patentanwälte OG

(56) Entgegenhaltungen:
- WO-A1-2008/003920
- WO-A2-2009/114803
- DE-U1- 20 213 196
- US-A- 5 463 377
- US-B1- 6 287 253

## Beschreibung

Die Erfindung betrifft eine Vorrichtung zur Kontrolle des Erreichens des korrekten Anpressdruckes von medizinischen Druckverbänden- und -bandagen, mit einer Sensoreinheit und einer Auslese- und Auswerteeinheit, wobei die Sensoreinheit und die Auslese- und Auswerteeinheit berührungslos miteinander in induktive Wirkverbindung bringbar sind.

In der medizinischen Kompressionstherapie werden dem Patienten Druckverbände bzw. Druckbandagen auf den zu behandelnden Extremitäten angelegt. Um den therapeutischen Erfolg sicherzustellen, ist es wesentlich, durch einen entsprechenden Bandagenzug einen bestimmten Minimaldruck auf die Extremität und insbesondere auf die entsprechenden Gefäße auszuüben. Aus Komfortgründen und aus Gründen der Hygiene ist es wünschenswert, den Druckverband von Zeit zu Zeit abzunehmen, beispielsweise um den betreffenden Körperteil waschen zu können. Um nach einem derartigen Abnehmen des Druckverbandes auch einem unerfahrenen Therapeuten oder sogar dem Patienten selbst das Anlegen des Druckverbandes mit dem erforderlichen Druck zu ermöglichen, sind im Stand der Technik Vorrichtungen zur Kontrolle des Erreichens des korrekten Anpressdruckes derartiger medizinischer Druckverbände bzw. Druckbandagen bekannt geworden. Bei diesen Systemen wird ein Sensor zwischen der Haut und Druckverband, d.h. zwischen der Haut und beispielsweise einer Bandage des Druckverbands eingelegt, woraufhin der Verband weiter angelegt bzw. festgezogen wird, wobei bei Erreichen des erforderlichen Anpressdrucks ein entsprechendes Signal aus der Sensoreinheit ausgelesen und angezeigt werden kann. Bei Systemen, bei welchen das Signal des Sensors mit Hilfe von Kabeln nach außen in die Auslese- und Auswerteeinheit geleitet wurde, verursachten diese Kabel häufig Druckstellen und entsprechende Beschwerden beim Patienten.

Um diesem Missstand zu begegnen, ist in der deutschen Gebrauchsmusterschrift DE 202 13 196 U1 eine Vorrichtung zur Messung und Überwachung von auf den menschlichen Körper einwirkenden lokalen Druckbelastungen bekannt geworden, bei welcher ein kapazitiver Drucksensor von einem Füllmedium in einer Hülle einer Sensoreinheit in Abhängigkeit vom angelegten Druck Signale lieferte, welche in der Sensoreinheit ausgewertet wurden. Die Signale konnten über eine Koppelspule kabellos von einer externen Auswerteschaltung ausgelesen werden, sodass die genannten Kabel entfallen konnten. Nachteilig war bei dieser Vorrichtung, dass der kapazitive Sensor relativ komplex aufgebaut war, und insbesondere, dass Schaltungseinheiten in der Sensoreinheit zu einer Telemetrieschaltung zusammengefasst waren, weswegen die Sensoreinheit relativ aufwendig und somit kostspielig war.

Auch die Veröffentlichung WO 2008/003920 A1 zeigt eine Vorrichtung gemäß der Präambel von Anspruch 1, wobei als Drucksensor eine Reihe von Druckschaltern offenbart ist, die bei verschiedenen Anpressdrücken schließen, und somit Abstufungen des Anpressdrucks detektieren können. Details der induktiven Signalübertragung sind nicht offenbart.

Es ist somit Aufgabe der vorliegenden Erfindung, eine Vorrichtung der eingangs genannten Art dahingehend weiterzubilden, dass die Sensoreinheiten überaus einfach, billig und in großer Zahl hergestellt werden können, um diese sodann ohne weiteres als Einmalprodukte an die Patienten abgeben zu können. Zur Lösung dieser Aufgabe ist eine Vorrichtung der eingangs genannten Art dahingehend weitergebildet, dass die Sensoreinheit von einer ersten Spule gebildet ist, welche durch einen mechanischen Druckschalter kurzschließbar ist, und dass die Auslese- und Auswerteinheit einen Schwingkreis mit einer zweiten Spule umfasst. Eine ähnliche Maßnahme offenbart das Dokument US 5463377 A für einen Flüssigkeitssensor, wobei die Sensoreinheit durch eine Spule gebildet ist, die bei Feuchtigkeit kurzgeschlossen wird. Bei einer solchen Vorrichtung ist die Sensoreinheit überaus einfach aufgebaut und besteht lediglich aus einer Spule und einem mechanischen Druckschalter, auf welchen der Druck des Druckverbandes bzw. der Druckbandage wirkt, wobei bei Erreichen eines bestimmten Druckes der Schalter schließt und dadurch die Spule kurzschließt. Der Schwingkreis in der Auslese- und Auswerteeinheit besitzt eine gewisse Eigenfrequenz, welche bei

Näherung der Spule der Sensoreinheit an eine andere Spule durch die induktive Leistungsaufnahme der Spule eine gewisse Variation erfährt. Wenn nun bei Erreichen des erforderlichen Drucks des Druckverbandes bzw. der Druckbandage der Kontakt am Druckschalter der Sensorspule geschlossen wird, so ändert die Spule der Sensoreinheit ihre Leistungsaufnahme, was zu der genannten Variation der Frequenz des Schwingkreises der Auslese- und Auswerteeinheit führt. Diese Variation kann mit Hilfe einer Auswerteschaltung in der Auslese- und Auswerteeinheit erfasst und dem Therapeuten bzw. dem Patienten angezeigt werden.

Um die Sensoreinheit der erfindungsgemäßen Vorrichtung besonders flach und somit besonders komfortabel für den Anwender zu gestalten, ist die erfindungsgemäße Vorrichtung mit Vorteil dahingehend weitergebildet, dass die erste Spule und/oder die zweite Spule als flache Spule in Form einer gedruckten Schaltung ausgebildet ist/sind. Die Realisierung von Spulen als flache Spulen in Form von gedruckten Schaltungen ist im Stand der Technik bestens bekannt, wobei die einzelnen Windungen der Spulen spiralförmig in einer Ebene nebeneinander zu liegen kommen.

Um einen dem therapeutischen Zweck entsprechenden Druckpunkt des Druckschalters exakt definieren zu können, ist die Vorrichtung in bevorzugter Weise dahingehend weitergebildet, dass der Druckschalter als Folienschalter ausgebildet ist. Solche Folienschalter bestehen im Wesentlichen aus einer Grundplatte und einer über der Grundplatte angeordneten kuppelförmigen Folie, auf welcher eine erste Kontaktfläche des Schalters angeordnet ist. Wenn nun Druck auf die kuppelförmige Folie ausgeübt wird, widersteht die Folie einer Deformierung aufgrund ihrer kuppelförmigen Geometrie bis zu einem gewissen Druck, wobei bei Überschreiten dieses Grenzdruckes die Kuppel plötzlich einklappt und der Kontakt mit einer zweiten oder mehreren Kontaktflächen geschlossen wird.

Um dem Anwender eine Kontrolle des Erreichens des entsprechenden Druckpunktes anzuzeigen, ist die Vorrichtung in einfacher weise dahingehend weitergebildet, dass die Auslese- und Auswerteeinheit Mittel zum Anzeigen der Variation der Frequenz des Schwingkreises aufweist, wobei die Vorrichtung mit Vorteil dahingehend weitergebildet ist, dass die Mittel zum Anzeigen der Variation der Frequenz des Schwingkreises von zumindest einer Kontrolleuchte, insbesondere LED gebildet sind.

Es ist weiters eine Aufgabe der vorliegenden Erfindung, einen medizinischen Druckverband bereitzustellen, welcher es gestattet, an verschiedenen Stellen des Druckverbandes das Erreichen eines bestimmten Druckes zu kontrollieren. Zur Lösung dieser Aufgabe ist ein medizinischer Druckverband mit wenigstens einer Sensoreinheit, wie in einem der Ansprüche 1 bis 5 beschrieben, ausgestattet. Die Sensoreinheiten werden dabei an den zu überwachenden Stellen im Verband angebracht und das Erreichen des entsprechenden Druckes an den jeweiligen Stellen durch separates Auslesen der Sensoreinheiten durch eine Auslese- und Auswerteeinheit kontrolliert.

Die Erfindung ermöglicht weiters ein Verfahren zur Kontrolle des Erreichens des korrekten Anpressdruckes von medizinischen Druckverbänden und -bandagen unter Verwendung der erfindungsgemäßen Vorrichtung, welches es auch unerfahrenen Anwendern und insbesondere dem Patienten selbst ermöglicht, den Druckverband bzw. die Druckbandage korrekt anzulegen. Zur Lösung dieser Aufgabe ist das Verfahren gekennzeichnet durch die folgenden Schritte:
Einlegen wenigstens einer Sensoreinheit in den Verband und Kalibrieren des Schwingkreises der Auslese- und Auswerteschaltung;
Herstellen der induktiven Wirkverbindung durch in-Deckung-Bringen der Spule der Auslese- und Auswerteinheit mit der Spule der Sensoreinheit;

Festziehen des Druckverbandes oder der Druckbandage bis die Auslese- und Auswerteschaltung das Erreichen des korrekten Anpressdrucks anzeigt.

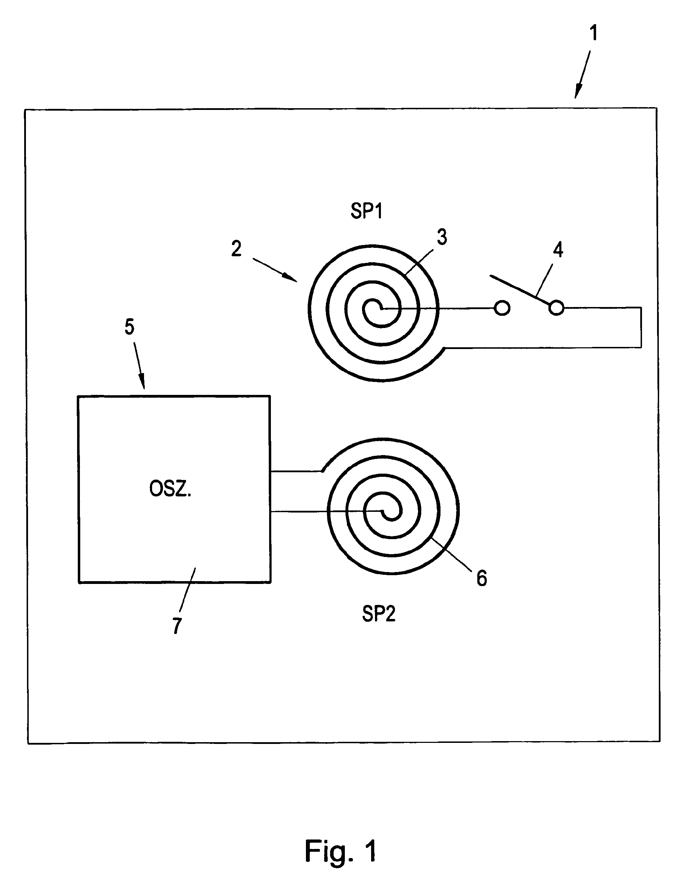

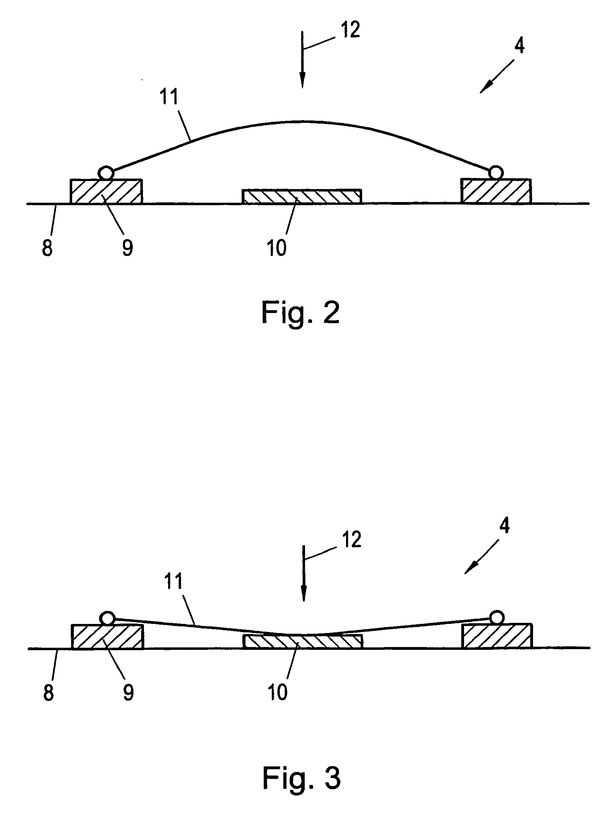

Die Erfindung wird nachfolgend anhand eines in der Zeichnung schematisch dargestellten Ausführungsbeispiels näher erläutert. In dieser zeigen Fig. 1 eine schematische Darstellung des Funktionsprinzips der erfindungsgemäßen Vorrichtung, Fig. 2 eine schematische Schnittdarstellung eines im Rahmen der Erfindung verwendbaren Druckschalters in nicht komprimiertem Zustand, und Fig. 3 eine schematische Schnittdarstellung des Druckschalters gemäß Fig. 2 in komprimiertem Zustand.

In Fig. 1 ist mit 1 eine Vorrichtung zur Kontrolle des Erreichens des korrekten Anpressdruckes von medizinischen Druckverbänden und -bandagen gemäß der vorliegenden Erfindung dargestellt. 2 bezeichnet eine Sensoreinheit, bestehend aus einer Spule 3 und einem Folienschalter 4, welcher ein mechanischer Druckschalter ist. Durch den Schalter 4 wird die Spule 3 bei Anliegen eines entsprechenden Auslösedruckes kurzgeschlossen, wobei dieser Zustand durch Verstimmung eines in die Nähe gebrachten Schwingkreises darstellbar ist. Mit 5 ist eine Auslese- und Auswerteeinheit bezeichnet, welche aus einem Schwingkreis mit einer Spule 6 und einer Schaltung 7 zum Bestimmen der Variation der Eigenfrequenz des Schwingkreises bezeichnet ist. Wenn die Spule 6 des Schwingkreises der Auslese- und Auswerteeinheit 5 mit der Spule 3 der Sensoreinheit 2 angenähert wird, erfolgt zunächst keine elektromagnetische Wechselwirkung. Wird bei Anlegen eines geeigneten Drucks der mit den Enden der Spule 3 verbundene Folienschalter 4 geschlossen, ändert sich die Leistungsaufnahme der Spule plötzlich, was zu einer Variation der Frequenz des Schwingkreises führt. Diese Änderung kann mit Hilfe der Auswerteschaltung 7 dem Anwender angezeigt werden.

Wie in Fig. 2 dargestellt, besteht der Folienschalter 4 aus einer Grundplatte 8, auf welche die Kontakte 9 und 10 aufgedruckt sind. Die kuppelförmige Folie 11 ist als konvexe und verformbare Kontaktfläche ausgebildet. Wird nun Druck in Richtung des Pfeils 12 auf die Folie 11 ausgeübt, so knickt diese bei einem gewissen Druck ein und der Kontakt zwischen den Kontaktflächen 9 und 10 wird geschlossen.

In Fig. 3 ist der Schalter 4 in komprimiertem Zustand dargestellt, wobei zu erkennen ist, dass die Kontaktfläche der Folie 11 die Kontakte 9 und 10 verbindet.

## Patentansprüche

1. Vorrichtung zur Kontrolle des Erreichens des korrekten Anpressdruckes von medizinischen Druckverbänden und -bandagen, mit einer Sensoreinheit und einer Auslese- und Auswerteeinheit, wobei die Sensoreinheit und die Auslese- und Auswerteeinheit berührungslos miteinander in induktive Wirkverbindung bringbar sind, **dadurch gekennzeichnet, dass** die sensoreinheit (2) von einer ersten Spule (3) gebildet ist, welche durch einen mechanischen Druckschalter (4) kurzschließbar ist, und dass die Auslese- und Auswerteeinheit (5) einen Schwingkreis mit einer zweiten Spule (6) umfasst.

2. Vorrichtung nach Anspruch 1, **dadurch gekennzeichnet, dass** die erste Spule (3) und/oder die zweite Spule (6) als flache Spule in Form einer gedruckten Schaltung ausgebildet ist/sind.

3. Vorrichtung nach Anspruch 1 oder 2, **dadurch gekennzeichnet, dass** der Druckschalter (4) als Folienschalter ausgebildet ist.

4. Vorrichtung nach Anspruch 1, 2 oder 3, **dadurch gekennzeichnet, dass** die Auslese- und Auswerteeinheit (5) Mittel zum Anzeigen der Variation der Frequenz des Schwingkreises aufweist.

5. Vorrichtung nach Anspruch 4, **dadurch gekennzeichnet, dass** die Mittel zum Anzeigen der Variation der Frequenz des Schwingkreises von zumindest einer Kontrolleuchte, insbesondere LED gebildet sind.

6. Medizinischer Druckverband mit wenigstens einer Sensoreinheit (2) wie in einem der Ansprüche 1 bild 5 beschrieben.

## Claims

1. A device for checking the attainment of the correct application pressure of medical compresses and bandages, including a sensor unit and a read-out and evaluation unit, wherein the sensor unit and the read-out and evaluation unit can be brought into inductive operative connection with each other in a contactless manner, **characterized in that** the sensor unit (2) is comprised of a first coil (3) capable of being shortcircuited by a mechanical pressure switch (4), and that the read-out and evaluation unit (5) comprises an oscillating circuit including a second coil (6).

2. A device according to claim 1, **characterized in that** the first coil (3) and/or the second coil (6) is/are configured as a flat coil in the form of a printed circuit.

3. A device according to claim 1 or 2, **characterized in that** the pressure switch (4) is configured as a membrane switch.

4. A device according to claim 1, 2 or 3, **characterized in that** the read-out and evaluation unit (5) comprises means for indicating the variation of the frequency of the oscillation circuit.

5. A device according to claim 4, **characterized in that** the means for indicating the variation of the frequency of the oscillation circuit are comprised of at least one indicator lamp, in particular LED.

6. A medical compress comprising at least one sensor unit (2) as described in any one of claims 1 to 5.

## Revendications

1. Dispositif pour contrôler l'application de la pression correcte par des pansements ou bandages compressifs médicaux, comportant une unité de détection et une unité de lecture et d'analyse, mais l'unité de détection et l'unité de lecture et d'analyse pouvant être amenées à coopérer sans contact de manière inductive l'une avec l'autre, **caractérisé en ce que** l'unité de détection (2) est formée par une première bobine (3) pouvant être court-circuitée par un interrupteur à pression (4) mécanique, et **en ce que** l'unité de lecture et d'analyse (5) comprend un circuit oscillant avec une deuxième bobine (6).

2. Dispositif selon la revendication 1, **caractérisé en ce que** la première bobine (3) et/ou la deuxième bobine (6) est/sont réalisée(s) sous la forme d'une bobine plate formant un circuit imprimé.

3. Dispositif selon la revendication 1 ou 2, **caractérisé en ce que** l'interrupteur à pression (4) est réalisé sous la forme d'un interrupteur à feuille.

4. Dispositif selon la revendication 1, 2 ou 3, **caractérisé en ce que** l'unité de lecture et d'analyse (5) comporte des moyens pour afficher la variation de la fréquence du circuit oscillant.

5. Dispositif selon la revendication 4, **caractérisé en ce que** les moyens pour afficher la variation de la fréquence du circuit oscillant sont formés par au moins un voyant de contrôle, en particulier une diode électroluminescente.

6. Pansement compressif médical comportant au moins une unité de détection (2), telle qu'elle est décrite dans l'une quelconque des revendications 1 à 5.
